# EUROPEAN PATENT APPLICATION

(11) **EP 1 561 438 A2**
(43) Date of publication of application: **10.08.2005**
(21) Application number: 05000063.7
(22) Date of filing: 04.01.2005
(51) Int. Cl.: A61F 2/46, A61B 19/00

(54) **Orthopaedic component inserter for use with a surgical navigation system**

(30) Priority: 03.02.2004 US 770963
(71) Applicant: Zimmer Technology, Inc., Chicago, Illinois 60606 (US)
(72) Inventor: Grimm, James E., Winona Lake IN 46590 (US); McGinley, Shawn E., Fort Wayne,IN 46814 (US)
(74) Representative: Manitz, Finsterwald & Partner GbR

(57) **Abstract**

The present invention provides an orthopaedic component inserter usable with a surgical navigation system.

## Description

The present invention relates to devices usable with surgical navigation systems. In particular, the present invention relates to an orthopaedic component inserter for inserting an orthopaedic component into a surgical site of a patient during orthopaedic surgery.

Many surgical procedures are now performed with surgical navigation systems in which sensors detect tracking elements attached in known relationship to an object in the surgical suite such as a surgical instrument, implant, or patient body part. The sensor information is fed to a computer that then triangulates the position of the tracking elements within the surgical navigation system coordinate system. Thus, the computer can resolve the position and orientation of the object and display the position and orientation for surgeon guidance. For example, the position and orientation can be shown superimposed on an image of the patient's anatomy obtained via X-ray, CT scan, ultrasound, or other imaging technology.

During orthopaedic surgery, orthopaedic components in the form of instruments to prepare a bone, provisional components to verify sizing, implant components and/or other suitable components are placed in a surgical site. These components often have a preferred position and orientation for optimal performance. An orthopaedic component inserter provides a mechanism to grip the orthopaedic component and extend it into the surgical site. The inserter may further provide a mechanism for aligning the orthopaedic component in a desired orientation.

The present invention provides an orthopaedic component inserter usable with a surgical navigation system.

In one aspect of the invention, an orthopaedic component inserter for inserting an orthopaedic component into a surgical site of a surgical patient while using a surgical navigation system includes a body having an orthopaedic component engagement end for releasably receiving the orthopaedic component in predetermined known relationship to the orthopaedic component engagement end. The inserter further includes a surgical navigation mechanism and a mechanical alignment mechanism.

In another aspect of the invention, an orthopaedic component inserter for inserting an orthopaedic component into a surgical site of a surgical patient's body while using a surgical navigation system includes a body having an orthopaedic component engagement portion for releasably receiving the orthopaedic component in predetermined known relationship to the orthopaedic component engagement portion. The inserter further includes first and second surgical navigation mechanisms. The second surgical navigation mechanism includes means for releasably connecting it to the orthopaedic component engagement portion of the body to permit the surgical navigation system to determine the location of the acetabular component engagement portion of the body relative to the first surgical navigation mechanism.

In another aspect of the invention, an acetabular component inserter for inserting an acetabular component into the acetabulum of a surgical patient's pelvis while using a surgical navigation system includes a shaft having an acetabular component engagement end for releasably receiving the acetabular component. The inserter further includes a first surgical navigation mechanism connected to the shaft. The surgical navigation mechanism is trackable by the surgical navigation system such that a computer can identify the position and orientation of the acetabular component attached to the acetabular component engagement end of the shaft.

In another aspect of the invention, an acetabular component inserter for inserting an acetabular component into the acetabulum of a surgical patient's pelvis while using a surgical navigation system includes a shaft having an acetabular component engagement end for releasably receiving the acetabular component. The inserter further includes a surgical navigation mechanism, a mechanical alignment mechanism, and means for alternatively connecting the alignment mechanisms to the shaft to permit the acetabular component inserter to be converted between mechanical and surgical navigation alignment configurations.

A method for inserting an orthopaedic component into a surgical site of a surgical patient in conjunction with a surgical navigation system includes: providing an orthopaedic component inserter having a body including an orthopaedic component engagement portion for releasably engaging an orthopaedic component; providing a surgical navigation alignment mechanism, the surgical navigation alignment mechanism being trackable by the surgical navigation system such that the surgical navigation system is able to identify the orientation of an orthopaedic component connected to the body; providing a mechanical alignment mechanism, the mechanical alignment mechanism having at least one feature visually alignable by a human user with a portion of the patient's anatomy to place an orthopaedic component connected to the body in a desired orientation relative to the surgical site; selecting one of the surgical navigation and mechanical alignment mechanisms; attaching the selected mechanism to the body; attaching an orthopaedic component to the body; inserting the orthopaedic component into the surgical site; and aligning the orthopaedic component using the selected alignment mechanism. Preferably, the surgical site comprises the patient's pelvis and the orthopaedic component comprises an acetabular component.

Another method for inserting an orthopaedic component into a surgical site of a surgical patient in conjunction with a surgical navigation system includes: providing an orthopaedic component inserter having a body including an orthopaedic component engagement portion for releasably engaging an orthopaedic component; providing a surgical navigation alignment mechanism, the surgical navigation alignment mechanism being trackable by the surgical navigation system such that the surgical navigation system is able to identify the orientation of an orthopaedic component connected to the body; connecting the surgical navigation alignment mechanism to the body; providing a calibration mechanism engageable with the orthopaedic component engagement portion of the body, the calibration mechanism being trackable by the surgical navigation system; engaging the calibration mechanism with the orthopaedic component engagement portion of the body; and using the surgical navigation system to identify the position and orientation of the orthopaedic component engagement portion of the body relative to the surgical navigation alignment mechanism. Preferably, the surgical site comprises the patient's pelvis and the orthopaedic component comprises an acetabular component. Preferably, the method further comprises: storing the relative position of the orthopaedic component engagement portion of the body in surgical navigation system memory; disengaging the calibration mechanism from the body; attaching an orthopaedic component to the orthopaedic component engaging portion of the body; and inserting the orthopaedic component into the surgical site while using the surgical navigation system to verify the position and orientation of the orthopaedic component. Preferably, the method further comprises: storing size information for a plurality of orthopaedic components in surgical navigation system memory; and using the stored relative position of the orthopaedic component engagement portion of the body and the stored size information to resolve the position and orientation of a particular orthopaedic component engaged with the body.

Various embodiments of the present invention will be discussed with reference to the appended drawings. These drawings depict only illustrative embodiments of the invention and are not to be considered limiting of its scope.

FIG. 1 is a perspective view of an illustrative orthopaedic component inserter according to the present invention including multiple alignment mechanisms and a removable calibration device;

FIGS. 2 and 3 are perspective views of the removable calibration device of FIG. 1;

FIG. 4 is a perspective view of an illustrative calibration block for use with the inserter of FIG. 1; and

FIG. 5 is a side cross sectional view of the inserter of FIG. 1 shown engaged with the calibration block of FIG. 4.

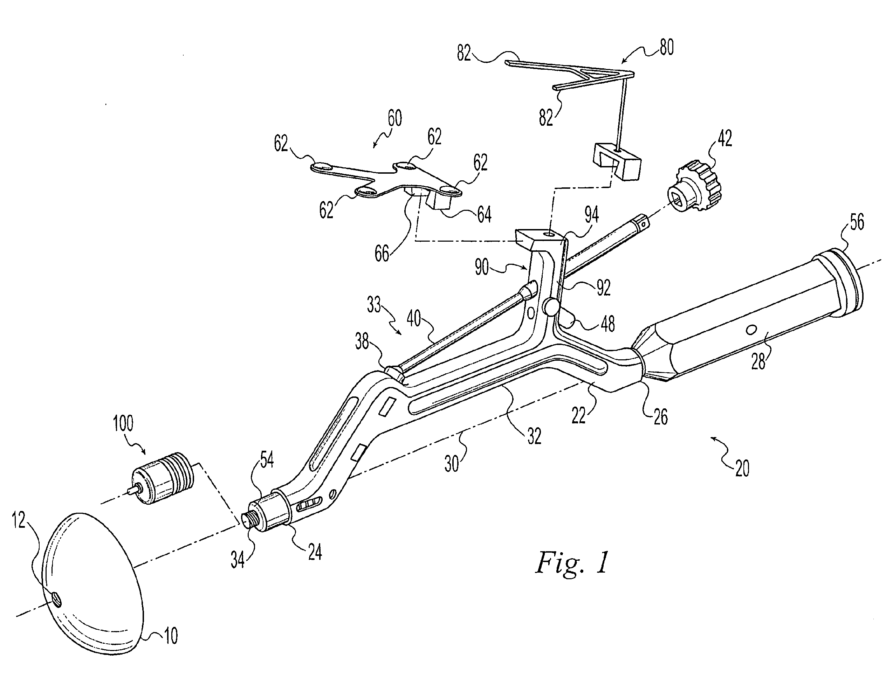

Embodiments of an orthopaedic component inserter may be configured to engage and position various orthopaedic components including cutting instruments, reaming instruments, provisional implants, implants, and/or other components for any suitable surgical site. Examples of surgical sites include hip joints, knee joints, vertebral joints, shoulder joints, elbow joints, ankle joints, digital joints of the hand and feet, fracture sites, tumor sites, and/or other suitable orthopaedic surgical sites. FIG. 1 depicts an illustrative orthopaedic component inserter 20 configured to engage an acetabular component and facilitate positioning the component in a patient's acetabulum and orienting it in a desired orientation. This illustrative inserter 20 is by way of example only and should not be considered limiting of the scope of the invention.

The illustrative inserter 20 is shown engaging an acetabular shell component 10 of a multi-part acetabular implant. However, the inserter 20 may be configured to engage other components such as an acetabular reaming instrument, a provisional acetabular implant, an acetabular bearing component of a multi-part acetabular implant, a unitary acetabular implant, and/or other suitable components. The illustrative inserter 20 is shown with a threaded engagement mechanism for engaging a threaded polar hole 12 of the acetabular shell component 10. However, the gender of the threaded connection may be reversed. Moreover, other mechanisms for engaging an acetabular component are contemplated such as a press fit engagement, snap fit engagement, clamping engagement, and/or other suitable engagements. Likewise, the engagement mechanism may engage the acetabular component interior and/or the acetabular component exterior via frictional and/or positive engagements. The engagement mechanism may engage a surface, hole, notch, groove, and/or other suitable feature located at the component pole, equator, and/or any intermediate position.

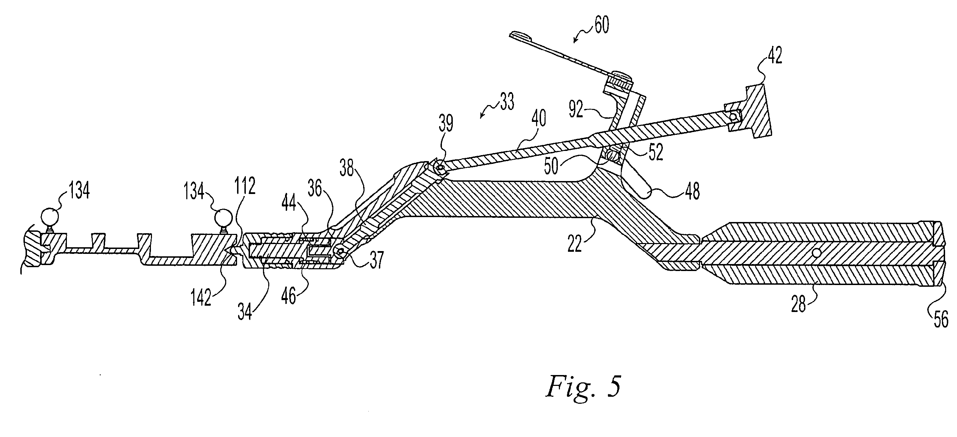

In the illustrative embodiment of FIG. 1, the inserter 20 includes a shaft 22 having an acetabular component engaging end 24 and a grip end 26 connected to a handle 28. The acetabular component engaging end 24 and handle 28 are collinear along an axis 30 so that insertion forces imparted to the handle 28 are transmitted in a straight line to the acetabular component 10 without any tendency to tip the acetabular component 10. The illustrative shaft 22 is offset in a middle portion 32 away from the axis 30 to provide clearance for the edge of the surgical wound. However, a straight shaft lying along axis 30 is also within the scope of the invention. The illustrative inserter 20 has an engagement mechanism 33 including a threaded stud 34 mounted axially in the acetabular component engaging end 24 of the shaft 22. The threaded stud 34 is engageable with a threaded polar hole 12 of the acetabular component 10 to secure the acetabular component 10 to the inserter 20. The engagement mechanism 33 further includes a series of links 36, 38, 40 (FIG. 5) connecting the threaded stud 34 to a rotating handle 42. The links 36, 38, 40 connect to one another via universal joints 37, 39 to permit torsional forces input to the rotating handle 42 to be transmitted through the shaft offset and to the threaded stud 34 to turn the threaded stud 34 into engagement with the polar hole 12 of the acetabular component 10. The links 36, 38, 40 connect to the threaded stud 34 via a splined joint 44 to transmit rotation to the threaded stud 34 while permitting axial translation of the threaded stud 34. A spring 46 biases the threaded stud 34 away from the acetabular component 10 to retract the threaded stud 34 from the polar hole 12 as the threaded stud 34 is disengaged from the acetabular component 10. A locking lever 48 is mounted on the shaft 22 for locking the engagement mechanism 33 to prevent unwanted loosening of the acetabular component from the threaded stud 34. The locking lever 48 connects to a cam 50 that is rotatable to press a cam follower 52 against one of the links 40 to prevent the threaded stud from rotating until the locking lever 48 is released. The acetabular component engaging end 24 of the shaft 22 includes an abutment surface 54 that presses against the acetabular component 10 as the threaded stud 34 is tightened to securely clamp the acetabular component 10 into engagement with the shaft 22. With the acetabular component 10 engaged with the shaft 22, the inserter 20 may be used to position the acetabular component 10 in the acetabulum. The handle 28 permits gripping the inserter 20 to direct axial forces to the acetabular component 10. In addition, a striking surface 56 at the end of the handle 28 allows the inserter to be impacted with a mallet to drive the acetabular component 10 into firm seated engagement with the acetabulum.

The inserter 20 includes multiple alignment mechanisms to aid in positioning and orienting the acetabular component 10. A surgical navigation mechanism 60 is trackable by a surgical navigation system such that a computer can identify when the acetabular component 10 is placed in a desired orientation relative to the acetabulum. A mechanical alignment mechanism 80 is visually alignable by the surgeon with a portion of the patient's anatomy to place the acetabular component 10 in a desired orientation relative to the acetabulum. In the illustrative embodiment of FIG. 1, the multiple alignment mechanisms 60, 80 are shown as being alternatively releasably connectable to the shaft 22 to permit the inserter 20 to be converted between surgical navigation and mechanical alignment configurations. However, the mechanisms 60, 80 may be combined into a single multipurpose mechanism releasably connectable to the shaft 22 and/or either one or both of the mechanisms 60, 80 may be permanently connected to the shaft 22.

The shaft 22 includes an alignment mechanism engagement portion 90 for receiving the alignment mechanisms 60, 80. In the illustrative embodiment, the engagement portion 90 includes a riser 92 with a dovetail mounting block 94. While the illustrative embodiment has depicted a dovetail engagement 94 of the alignment mechanisms 60, 80 directly to a portion of the shaft 22, other connections are contemplated. For example, the alignment mechanisms 60, 80 may be bolted, welded, clamped, and/or otherwise attached directly or indirectly to the shaft 22. The alignment mechanisms 60, 80 may be indirectly connected to the shaft 22 by being connected to the handle 28, acetabular component engagement end 24, and/or other portion of the inserter 20. As long as the alignment mechanism 60, 80 is linked in a known relationship to the acetabular component 10, the alignment mechanism 60, 80 may be used to position the acetabular component 10.

The surgical navigation alignment mechanism 60 includes an array of tracking elements 62 and a mounting portion 64 for engaging the inserter 20 in a predetermined orientation. In the illustrative embodiment, the mounting portion 64 includes a female dovetail 66 for engaging the dovetail mounting block 94. The tracking elements 62 are detectable by the surgical navigation system such that the three dimensional position of the tracking elements can be related to a surgical navigation coordinate system. For example, the surgical navigation system may include multiple sensors at known locations that feed tracking element 62 position information to a computer. The computer may then use the position information from the multiple sensors to triangulate the position of each tracking element within the surgical navigation coordinate system. The surgical navigation system can then determine the position and orientation of the inserter 20 by detecting the position and orientation of the tracking elements and resolving the position and orientation of the inserter 20 from the known relationship between the tracking elements and the inserter 20.

The tracking elements may be detectable by imaging, acoustically, electromagnetically, or by other suitable detection means. Furthermore, the tracking elements may be active or passive. Examples of active tracking elements may include light emitting diodes in an imaging system, ultrasonic emitters in an acoustic system, and electromagnetic field emitters in an electromagnetic system. Examples of passive tracking elements may include elements with reflective surfaces.

The mechanical alignment mechanism 80 includes at least one feature visually alignable by a human user with a portion of the patient's anatomy to place the acetabular component 10 in a desired orientation. For example, the illustrative mechanical alignment mechanism 80 includes a pair of arms 82 that angle outwardly from the axis 30 at an angle of approximately 45°. One arm 82 is used for a left hip and the other is used for a right hip. The riser 92 is angled away from normal to the shaft axis by approximately 20°. Thus, by positioning one of the arms 82 parallel to the patient's body, the acetabular component 10 is placed in a desired 45° abduction and 20° forward flexion. Other suitable angles may be used as is appropriate for the implant, surgical technique, and anatomical reference. By providing both surgical navigation and mechanical alignment mechanisms 60, 80 usable with a single inserter, the surgeon is allowed to choose the most appropriate alignment mechanism for a given situation without having to change instruments.

When the surgical navigation alignment mechanism 60 is used, the inserter may be calibrated to the surgical navigation system. Due to manufacturing tolerances, variations in intraoperative assembly and disassembly, post operative cleaning, and/or other factors, the precise relationship between the surgical navigation alignment mechanism 60 and the acetabular component engaging end 24 may not be known. By engaging a calibration array of tracking elements with the acetabular component engaging end 24 in a predetermined known relationship, the surgical navigation system can detect the surgical navigation alignment mechanism 60 and the calibration array and determine the relationship between the surgical navigation alignment mechanism 60 and the acetabular component engaging end 24. This relationship may then be stored in the navigation system's memory and used to resolve the position and location of acetabular components 10 connected to the inserter 20. By registering the location of the acetabular component engaging end 24 rather than the location of a particular acetabular component 10 attached to the inserter 20, the inserter can be used with acetabular components 10 of any geometry without the need to recalibrate for each acetabular component 10. The geometry of each potential acetabular component 10 is stored in system memory and recalled as needed to resolve the position and orientation of the acetabular component 10 when it is connected to the acetabular component engaging end 24. The system can accurately resolve the position and orientation of each acetabular component 10 due to the use of simple, precise calibration mechanisms and attachment mechanisms that permit precise manufacturing and reproducible assembly of the calibration mechanism components, acetabular components, and engagement mechanisms. For example, by engaging a calibration array with the acetabular component engagement end 24 of the shaft 22, the position of the abutment surface 54 and axis 30 relative to the calibration array may be accurately determined. This calibration step negates any variation in manufacturing or assembly of the instrument between the surgical navigation mechanism 60 and the acetabular component engagement end 24. The abutment surface 54 may be a simple shape such as a the end of a cylinder (as shown) to permit precise manufacturing of the abutment surface 54 with relative ease. The acetabular components 10 may likewise comprise simple geometric shapes such as hemispheres that permit precise manufacturing with relative ease. By threading the stud 34 into the polar hole 12 of the acetabular component 10 and drawing the acetabular component 10 into contact with the abutment surface 54, the acetabular component 10 can be precisely and reproducibly attached to the inserter 20. Then, for each known acetabular component 10 geometry, the surgical navigation system can resolve the position and orientation of the acetabular component 10 with little error due to manufacturing tolerance or variation in assembly.

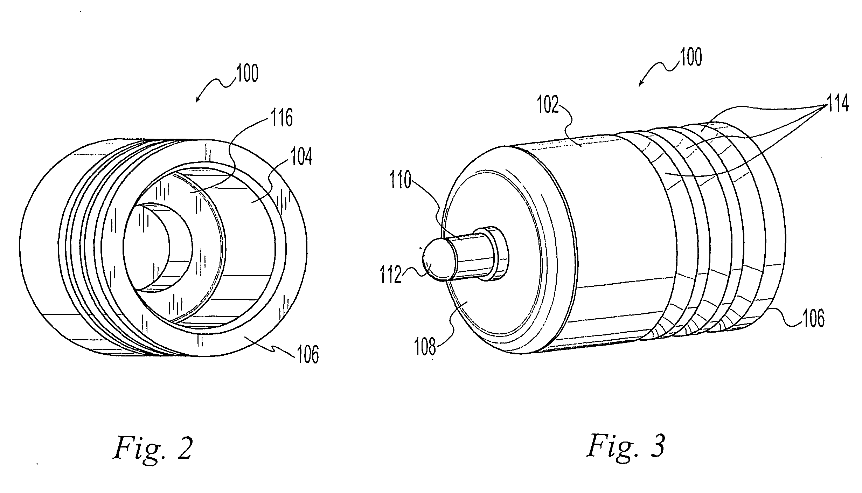

In the illustrative embodiment of FIG. 1, a calibration tip 100 is provided as part of an engagement mechanism for engaging a reference array. FIGS. 2 and 3 illustrate the calibration tip 100 in greater detail. The calibration tip 100 includes a hollow cylindrical body having an exterior surface 102, an interior surface 104, an inserter engaging end 106, and a calibration array engaging end 108. A calibration array engaging portion in the form of a cylindrical projection 110 extends from the calibration array engaging end 108 and terminates in a hemispherical tip 112. Finger grip grooves 114 formed in the exterior surface 102 facilitate gripping and handling the calibration tip 100. The interior of the calibration tip 100 opens outwardly at the inserter engaging end 106 to receive the acetabular component engaging end 24 of the inserter 20. An interior end surface 116 at least partially closes the calibration array engaging end 108. The calibration tip 100 fits over the acetabular component engaging end 24 of the inserter 20 in coaxial arrangement with the interior end surface 116 in contact with the abutment surface 54. The simple cylindrical configuration of the calibration tip 100 permits it to be easily manufactured to precise dimensions.

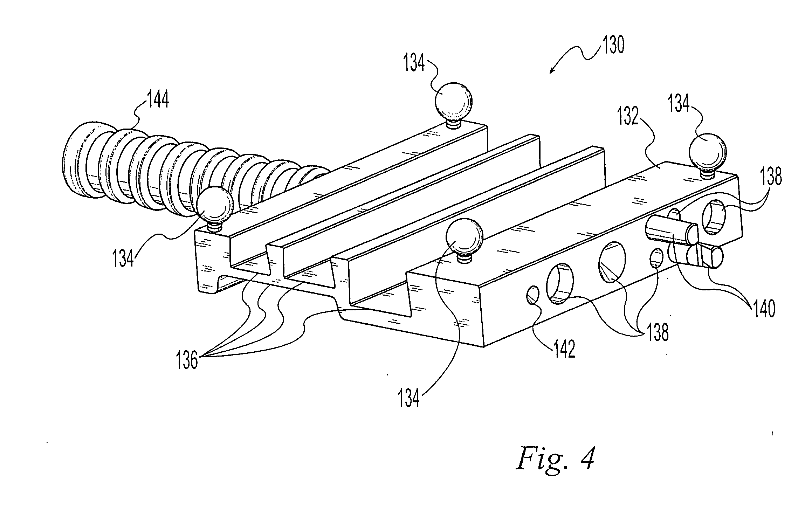

FIG. 4 shows an illustrative calibration block 130. The calibration block 130 includes a body 132 supporting an array of tracking elements 134 trackable by the surgical navigation system. The body 132 may include various slots 136, holes 138, pins 140, and other engagement features in known relationship to the array of tracking elements 134. These engagement features permit instruments needing calibration to be engaged with the calibration block in known relationship to permit calibration. In particular, a conical hole 142 is provided to engage the hemispherical tip 112 of the cylindrical projection 110 of the calibration tip 100. A handle 144 is provided to facilitate gripping the calibration block 130.

In use, the user determines whether it is desirable to use the surgical navigation or mechanical alignment mechanism 60, 80. If the mechanisms are provided as separate modular pieces, as in the illustrative embodiment, the user would then attach the appropriate mechanism to the inserter. If mechanical alignment is being used, an acetabular component 10 is attached to the acetabular component engaging end 24 of the inserter 20. In the illustrative embodiment, the threaded stud 34 is threaded into the polar hole 12 of the acetabular component 10 and the acetabular component 10 is drawn against the abutment portion 54 by rotating the acetabular component engagement mechanism 33. The engagement mechanism 33 is locked by rotating the locking lever 48 to drive the cam follower 52 against one of the rotating links 40 and wedge it against rotation. The acetabular component 10 is then positioned in the acetabulum by inserting the assembly into the surgical wound. A feature of the mechanical alignment mechanism 80 is visually aligned with a portion of the patient's anatomy to orient the acetabular component 10 in a desired orientation. For example, one of the arms 82 may be aligned parallel to the patient's body to place the acetabular component 10 in a desired abduction and forward flexion orientation. The acetabular component 10 may be further seated by impacting the striking surface 56 at the end of the handle 28. Once the acetabular component 10 is oriented and seated, the locking lever 48 is released and the engagement mechanism 33 is rotated to disengage the threaded stud 34 from the acetabular component 10. As the stud 34 turns, the spring 46 biases it out of engagement with the acetabular component 10. Once the inserter 20 is disengaged from the acetabular component 10, the inserter is removed and the procedure may proceed with implantation of other components, closing of the wound, and/or other required steps.

If the surgical navigation alignment mechanism 60 is being used, a calibration array may be engaged with the acetabular component engaging end 24 of the inserter 20 to calibrate the inserter 20. For example, the calibration tip 100 may be attached to the acetabular component engaging end 24 of the inserter 20 and then engaged with the calibration block 130. Pressing the hemispherical tip 112 of the calibration tip 100 into the conical hole 142 of the calibration block 130 will cause the hemispherical tip 112 to self-center on the conical hole 142 axis and insert to a predetermined distance. Thus the tip 112 will be at a precise, known location relative to the tracking elements 134 of the calibration block. If the surgical navigation system compares the positions of the calibration block tracking elements and the alignment mechanism 60 tracking elements 62 it can accurately resolve the location of the abutment surface 54 and axis 30. By storing the nominal geometry of the inserter 20 and alignment mechanism 60 in memory, the system can use the nominal geometry to predict where the tip 112 should be. By comparing this predicted location of the tip 112 to the actual location of the conical hole 142 in the calibration block 130, the system can determine not only the actual location of the tip 112 but also the actual orientation of the axis 30. Thus the system may positively determine the abutment surface 54 location and orientation. With this information and the geometry of the various acetabular components 10 to be used with the inserter 20, the navigation system can accurately determine the position and orientation of acetabular components 10 attached to the inserter 20. With the instrument calibrated, an acetabular component 10 is attached to the inserter 20 and then placed in the acetabulum. The surgical navigation system provides feedback on the position and orientation of the acetabular component 10. For example, the surgical navigation system may superimpose a graphical representation of the acetabular component 10 on X-ray, CT, or other medical image data and display the composite image on a computer monitor. When the acetabular component 10 is in the desired position and orientation as indicated by the surgical navigation system, the procedure may then be completed as described above with reference to the manual alignment mechanism.

The illustrative embodiment has shown one mechanism for engaging a calibration array. However, other mechanisms are contemplated and fall within the scope of the invention. For example, while the intermediate calibration tip 100 provides convenience by snapping over the acetabular component engaging end 24 of the inserter 20 and being self centering on the calibration block 130, it may be omitted and the calibration array may be engaged directly with the inserter 20. For example, the calibration block 130 may include an opening for engaging the acetabular component engaging end 24 of the shaft 22. Likewise, in the illustrative embodiment, the surgical navigation and mechanical alignment mechanisms 60, 80 are depicted as being used alternatively. However, they may be simultaneously attached to the inserter 20 and used together. For example, the surgical navigation mechanism 60 may be used for precise placement while the mechanical alignment mechanism may be used as a gross verification that the navigation system appears to be working properly.

Although embodiments of an orthopaedic component inserter and its use have been described and illustrated in detail, it is to be understood that the same is intended by way of illustration and example only and is not to be taken by way of limitation. Accordingly, variations in and modifications to the inserter and its use will be apparent to those of ordinary skill in the art, and the following claims are intended to cover all such modifications and equivalents.

## Claims

1. An orthopaedic component inserter for inserting an orthopaedic component into a surgical site of a surgical patient while using a surgical navigation system, the inserter comprising:
a body having an orthopaedic component engagement end for releasably receiving the orthopaedic component in predetermined known relationship to the orthopaedic component engagement end;
a surgical navigation mechanism having means for releasably connecting to the body in a fixed relationship to the body, the surgical navigation mechanism being trackable by the surgical navigation system such that a computer can identify the position and orientation of the orthopaedic component attached to the orthopaedic component engagement end of the body; and
a mechanical alignment mechanism having means for releasably connecting to the body in a fixed relationship to the body, the mechanical alignment mechanism having at least one feature visually alignable by a human user with a portion of the patient's anatomy to place the orthopaedic component in a desired orientation relative to the surgical site.

2. An orthopaedic component inserter for inserting an orthopaedic component into a surgical site of a surgical patient's body while using a surgical navigation system, the inserter comprising:
a body having an orthopaedic component engagement portion for releasably receiving the orthopaedic component in predetermined known relationship to the orthopaedic component engagement portion;
a first surgical navigation mechanism having means for releasably connecting to the body in a fixed relationship to the body, the surgical navigation mechanism being trackable by the surgical navigation system such that a computer can identify the position and orientation of the orthopaedic component attached to the orthopaedic component engagement portion of the body; and
a second surgical navigation mechanism having means for releasably connecting to the orthopaedic component engagement portion of the body to permit the surgical navigation system to determine the location of the orthopaedic component engagement portion of the body relative to the first surgical navigation mechanism.

3. An acetabular component inserter for inserting an acetabular component into the acetabulum of a surgical patient's pelvis while using a surgical navigation system, the inserter comprising:
a shaft having an acetabular component engagement end for releasably receiving the acetabular component in predetermined known relationship to the acetabular component engagement end; and
a first surgical navigation mechanism having means for releasably connecting to the shaft in a fixed relationship to the shaft, the surgical navigation mechanism being trackable by the surgical navigation system such that a computer can identify the position and orientation of the acetabular component attached to the acetabular component engagement end of the shaft.

4. The acetabular component inserter of claim 3 further comprising:
a mechanical alignment mechanism having means for releasably connecting to the shaft in a fixed relationship to the shaft, the mechanical alignment mechanism having at least one feature visually alignable by a human user with a portion of the patient's anatomy to place the acetabular component in a desired orientation relative to the acetabulum.

5. The acetabular component inserter of claim 3 or 4 further comprising a second surgical navigation mechanism having means for releasably connecting to the acetabular component engagement end of the shaft to permit the surgical navigation system to determine the location of the acetabular component engagement end of the shaft relative to the first surgical navigation mechanism.

6. The acetabular component inserter of claim 5 wherein the second surgical navigation mechanism is mounted on a calibration block having means for releasably engaging the acetabular component inserter.

7. The acetabular component inserter of claim 6 further comprising a calibration tip having means for releasably connecting to the acetabular component engagement end of the shaft, the calibration tip including means for engaging the calibration block.

8. The acetabular component inserter of claim 7 wherein the acetabular component engagement end of the shaft includes a cylindrical portion and an end surface and the calibration tip includes a cylindrical bore sized to fit closely around the cylindrical portion and an internal end wall that abuts the end surface of the acetabular component engagement end to locate the calibration tip in a predetermined position relative to the acetabular component engagement end.

9. The acetabular component inserter of claim 7 or 8 wherein the calibration tip includes a hemispherical projection and the calibration block includes a conical depression for engaging the spherical projection, the calibration tip being self-centering within the conical depression.

10. An acetabular component inserter for inserting an acetabular component into the acetabulum of a surgical patient's pelvis while using a surgical navigation system, the inserter comprising:
a shaft having an acetabular component engagement end for releasably receiving the acetabular component;
a surgical navigation mechanism, the surgical navigation mechanism being trackable by the surgical navigation system such that a computer can identify the position and orientation of the acetabular component attached to the acetabular component engagement end of the shaft;
a mechanical alignment mechanism, the mechanical alignment mechanism having at least one feature visually alignable by a human user with a portion of the patient's anatomy to place the shaft in a desired orientation relative to the acetabulum; and
means for alternatively connecting the alignment mechanisms to the shaft to permit the acetabular component inserter to be converted between mechanical and surgical navigation alignment configurations.
